# EUROPÄISCHE PATENTSCHRIFT

(11) **EP 2 555 872 B1**
(45) Veröffentlichungstag und Bekanntmachung des Hinweises auf die Patenterteilung: **11.03.2020**
(21) Anmeldenummer: 11716859.1
(22) Anmeldetag: 08.04.2011
(51) Int. Cl.: B01L 7/00, B01L 3/00

(54) **VORRICHTUNG ZUM NACHWEIS VON NUKLEINSÄUREN**
DEVICE FOR DETECTING NUCLEIC ACIDS
DISPOSITIF POUR LA DÉTECTION D'ACIDES NUCLÉIQUES

(30) Priorität: 08.04.2010 DE 102010003782
(43) Veröffentlichungstag der Anmeldung: 13.02.2013
(73) Patentinhaber: AJ Innuscreen GmbH, 13125 Berlin (DE)
(72) Erfinder: HILLEBRAND, Timo, 15366 Hoppegarten (DE); KNIPPSCHILD, Claus, 07743 Jena (DE); GRASER, Elmara, 13086 Berlin (DE)
(74) Vertreter: Wehlan, Martin
(86) Internationale Anmeldenummer: PCT/EP2011/055524
(87) Internationale Veröffentlichungsnummer: WO 2011/124688

(56) Entgegenhaltungen:
- EP-A1- 2 071 026
- EP-A1- 2 163 306
- WO-A1-97/27324
- DE-A1-102006 036 171
- DE-A1-102007 062 441
- US-A1- 2004 191 896

## Beschreibung

Gegenstand der Erfindung ist eine Vorrichtung, die es erlaubt, eine Zielnukleinsäure in einem homogenen Ansatz mit zwei unterschiedlichen Detektionsformaten nachzuweisen. Die Vorrichtung kann insbesondere für die mobile Gendiagnostik unter Feld-Bedingungen eingesetzt werden.

### Stand der Technik

Die Untersuchung diagnostisch relevanter biologischer Proben wie Serum, Plasma, Blut, Tupferproben oder Organabriebe zum Nachweis infektiöser Erreger hat in den letzten Jahren enorm an Bedeutung gewonnen. Virusinfektionen wie HIV, HCV oder HBV sind weltweit auf dem Vormarsch. Darüber hinaus sind auch bakterielle Infektionen wieder auf dem Vormarsch, u.a. auch als Ergebnis beginnender klimatischer Veränderungen. Das Auftreten neuer, tödlich verlaufender Infektionskrankheiten mit einem extrem hohen Infektiositätspotential (SARS, Vogelgrippe) zeigt immer deutlicher, dass eine schnelle und vor Ort durchführbare Diagnostik entscheidend für die Verhinderung von Epidemien sein wird. Darüber hinaus spielen einfach handhabbare und relativ preiswerte diagnostische Systeme, vor allem für Entwicklungsländer, ebenfalls eine bedeutende Rolle bei der Bekämpfung der Ausbreitung von Infektionskrankheiten. Die derzeitig vor allem für die Detektion viraler Infektionskrankheiten (HIV, HCV, HBV, Dengue, u.a.m.) eingesetzten Tests basieren mehrheitlich auf der Durchführung von REAL-TIME-PCR's. Diese Tests sind an extrem teure gerätetechnische Voraussetzungen gebunden sowie auch an teure Reagenzien. Die Durchführung dieser Methoden kann nur durch geschultes Fachpersonal in Speziallaboratorien erfolgen.

Ein weiterer Schwerpunkt betrifft die Durchführung von diagnostischen Tests in Bezug auf die Früherkennung von terroristischen Anschlägen (Pocken, Pest, Anthrax). Gerade bei diesen Anwendungsfeldern ist eine exakte Ergebnisfindung extrem wichtig, da von einem solchen Ergebnisse eine Vielzahl von weitreichenden Folgemaßnahmen für den Schutz der Bevölkerung eingeleitet wird. Zurzeit Verwendung findende diagnostische Tests (teilweise auch vor-Ort realisierbar) benötigen deshalb immer eine Bestätigungsuntersuchung auf einer zweiten Detektionsplattform (z.B. erste Diagnostik des Erregers mittels RealTime PCR und Bestätigung des diagnostischen Ergebnisses durch Auftragen des Amplifikationsansatzes auf ein Agarosegel). In diesem Kontext, ist eine wirkliche schnelle vor-Ort-Detektion nicht vollständig nötig, da die Bestätigungsuntersuchung auf einer zweiten Detektionsplattform kein Bestandteil von zur Zeit existierenden Systemlösungen ist (z.B. mobile LightCycler Detektion bietet nicht die Möglichkeit einer zweiten Bestätigungsdetektion).

Zum Stand der Technik gehört die US-Patentschrift 6,565,815 B1. Die in diesem Dokument beschriebene optische Einheit (Detektionseinheit) sieht eine Lichteinbringung vor, die seitlich von unten erfolgt und ist räumlich von der Heizeinheit getrennt ist. Die Reaktionskontrolle erfolgt hierbei nur auf einem optischen Weg. Eine weitere Produktkontrolle, wie immunologische Detektion mittels Lateral-Flow-Streifen ist nicht vorgesehen. Der Aufbau der Kartusche (Consumable) umfasst lichtreflektierende Wände, sowie eingebrachte optische Linsen. Hierbei werden mehrere Materialien für die Herstellung der Reaktionskammer benutzt. Der Strahlengang erfolgt durch zwei optische Fenster in der Reaktionskammer, die in einem Winkel von 90° zueinander liegen. Eine Absorptionsmessung ist hierbei nicht möglich, da keine Definition der Länge des Strahlenweges möglich ist. Der Anpressdruck der Probenblöcke in US 6,565,815 B1 wird nicht mittels einer Schraubenfeder realisiert. Die optische Einheit ist nicht unmittelbar am Probenblock justiert.

Ebenfalls Stand der Technik ist das Dokument DE 10 2006 036 171 A1. Dort wird eine Anordnung und ein Verfahren zur schnellen PCR mit mehrkanaliger Fluoreszenzmessung beschrieben. Die beschriebene Apparatur ist speziell auf 96er MTP (Mikrotiterplatte) ausgelegt. Der Reaktionsraum in DE 10 2006 036 171 A1 wird nicht durch die Heizelemente definiert, sondern ist durch die MTP vorgegeben. Aufgrund der Schichtdicke der Kavitätenwandung sind leistungsstarke Heizelemente (thermische Effizienz) unerlässlich und stehen somit einer Miniaturisierung bzw. der Mobilität (Spannungsversorgung) entgegen. Die Heizelemente befinden sich unter den Kavitäten, bzw. über ihnen (Deckelheizung). Der Reaktionsraum ist nicht senkrecht zu den Probeblöcken angeordnet. Die Einleitung des Lichtes erfolgt von oben (nicht seitlich) und nicht unmittelbar in die Probe, sondern in die Kavität, die ein undefiniertes Volumen an Luftüberstand aufweist. Des weiteren erfolgt sowohl die Einkopplung des Lichtes in die Probe, als auch dessen Auskopplung über die gleiche Lichtleitfaser. Eine Absorptionsmessung ist aufgrund der undefinierten Weglänge des Lichts nicht möglich. Aufgrund der längeren Wege des Lichtes in der Probe und die ungleich größeren Kavitäten sind auch optische Linsen nötig. Die beschriebene Anordnung stellt (auch aufgrund ihrer Mehrkanal-Fluoreszenzmessung und dem Fehlen einer internen Spannungsquelle) kein geeignetes Produkt zur Betriebnahme "im Feld" dar.

Ferner werden weitere Vorrichtungen zum Nachweis von Nukleinsäuren umfassend eine Reaktionskartusche, die einen Basiskörper und eine den Basiskörper abschließende Folie enthält, und eine optische Detektionseinheit in den Druckschriften US2004/191896A1, EP2071026A1 und WO7/27324A1 beschrieben.

Ziel der vorliegenden Erfindung war es deshalb, eine Detektionssystem bereitzustellen, welches einfach durchführbar ist (auch durch Nichtspezialisten), schnell ein diagnostisches Ergebnis liefert und auch vor Ort durchführbar sein sollte. Das Nachweissystem sollte es ermöglichen, zwei Detektionsmöglichkeiten zu realisieren, um das erste diagnostische Ergebnis zu verifizieren.

In den Druckschriften DE102007062441A1 und WO2009/080817A2 werden Gerätesysteme beschrieben, welche eine mobile Nukleinsäureisolierung aus beliebigen komplexen biologischen Proben sowie eine mobile Detektion von Nukleinsäuren vor Ort ermöglichen. Diese Gerätesysteme sind mittlerweile am Markt verfügbar und erlauben die Durchführung der Nukleinsäureisolierung und eine nachfolgende Detektion spezifischen Nukleinsäuren.

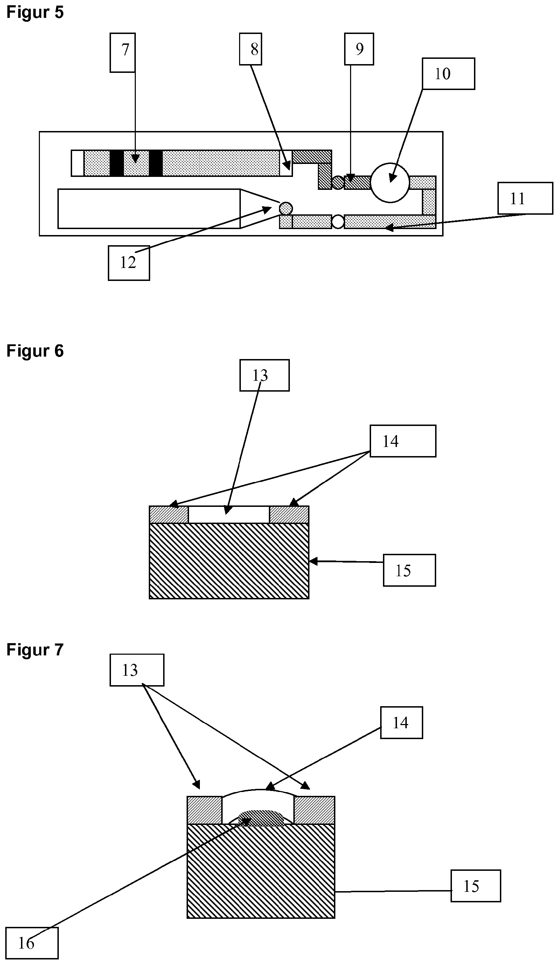

Kernstück der Detektionseinheit ist eine Reaktionskartusche, welche nach Beladung mit Amplifikationsreagenzien und zu untersuchender Probennukleinsäure die Durchführung einer extrem schnellen Amplifikationsreaktion ermöglicht. Der Nachweis der spezifischen Targetnukleinsäure erfolgt nachfolgend dadurch, dass der amplifizierte Reaktionsansatz auf einen nachgeschalteten Lateral Flow Teststreifen überführt wird. Auf dem Teststreifen erfolgt die finale kolorimetrische Detektion (Figur 5). Das Gesamtsystem arbeitet optional im Batteriebetrieb und ist damit auch unter Feldbedingungen einsetzbar.

Die auf dem Teststreifen ablaufende Nachweisreaktion ist ebenfalls in den Druckschriften DE102007062441A1 und WO2009/080817A2 offenbart. Es handelt sich um die Kombination einer Amplifikations- und Hybridisierungsreaktion und der nachfolgenden Detektion des Produktes auf einem immunologischen Teststreifen.

Das erfindungsgemäße Verfahren basiert auf der Verwendung der in den o.g. Druckschriften offenbarten Gerätesysteme und stellt eine deutlich verbesserte Variante dar.

Überraschenderweise zeigte sich, dass es möglich ist, eine duale Detektion einer Zielnukleinsäure in einer verbesserten Reaktionskartusche durchzuführen und ein solches Testsystem damit auch batteriebetrieben unter Feldbedingungen einzusetzen. Mit dem erfindungsgemäßen dualen Testsystem ist es erstmalig möglich, auch unter Feldbedingungen ein diagnostisches Ergebnis mittels eines zweiten Testformates zu verifizieren. Ein weiterer wesentlicher Vorteil ist dabei dadurch gegeben, dass beide Tests in einer neuartigen Reaktionskartusche ablaufen können und das die Reaktionskartusche eine homogene Testdurchführung beider Nachweisreaktionen ermöglicht, d.h. beide Nachweisreaktionen laufen so ab, dass zu keiner Zeit ein Öffnen der Reaktionskartusche notwendig ist. Dies bedeutet einen enormen Vorteil, da Amplifikationsprodukte somit nie in Kontakt mit der Umgebung gelangen und Kontaminationsrisiken damit nicht gegeben sind. (Im Gegensatz dazu sind Kontaminationen nach z.B. LightCycler Detektion möglich, wenn nachfolgend das Amplifikationsprodukt zur Bestätigung auf ein Agarosegel aufgetragen und aufgetrennt wird).

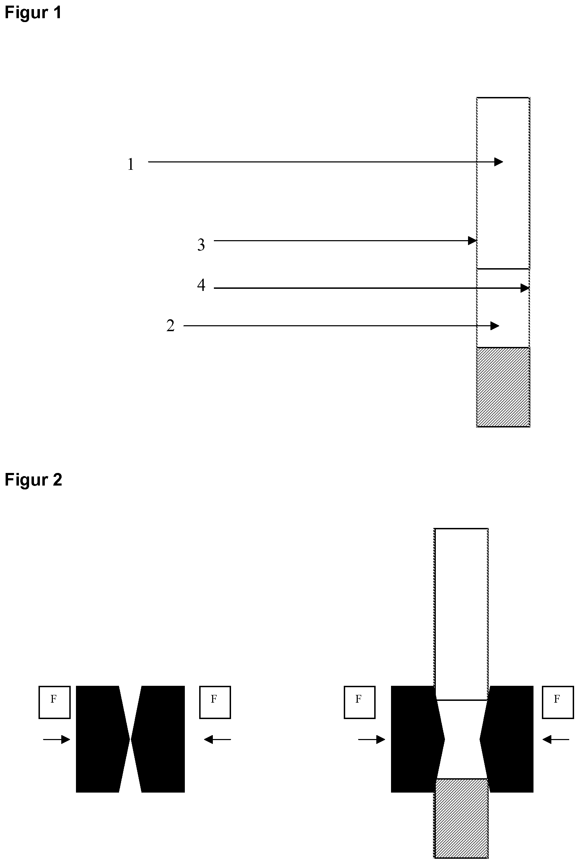

Die erfindungsgemäße Reaktionskartusche besteht vorzugsweise aus einem Basiskörper und mindestens einer den Basiskörper abschließenden Folie, welche mindestens eine nicht mit dem Basiskörper verbundene Fläche aufweist (Figur 1 und Figur 6 : Ausschnitt Reaktionsraum mit Folien). Die nicht verbundene Fläche der Folie zum Basisträger kann hierbei eine Reaktionskammer ausbilden (Figur 1). Ferner kann eine nicht verbundene Fläche auf dem Basisträger zum Volumentransport dienen (Figur 6) . Die nicht verbundenen Flächen können somit mehrere Volumen mit dem Basiskörper bilden, welche zum Beispiel zwei Reaktionskammern bilden und zum Volumentransport dienen. Dabei dient eine erste Reaktionskammer der erfindungsgemäßen optischen Detektion und die zweite Reaktionskammer ermöglicht eine immunologische Detektion.

Dazu enthält die zweite Reaktionskammer einen Lateral Flow Teststreifen. Die erfindungsgemäße Reaktionskartusche ermöglicht damit erstmalig eine duale Detektion auf zwei verschiedenen Detektionsprinzipien und erlaubt dadurch eine zweite, unabhängige Verifizierungsreaktion.

Die Kombination der beiden Detektionsformate wird wie folgt erfindungsgemäß realisiert.

Das erfindungsgemäße duale Detektionsverfahren basiert auf einer matrizenabhängigen DNA-Neusynthese mit einem Hybridisierungsschritt. Die erfindungsgemäße Auswahl der Markierungen der an den Reaktionen beteiligten Oligonukleotide erlaubt sowohl eine reaktionsabhängige Fluoreszenzmessung und damit verbundene numerische und ggf. quantitative Auswertung (optische Detektion), als auch eine nachfolgende immunologische Detektion in Form der Visualisierung der Reaktion z.B. auf einem Lateral-Flow-Streifen.

Erfindungsgemäß liegen die Probenblöcke bei nicht eingesetzter Kartusche aufeinander. Der Anpressdruck der Probenblöcke wird mittels einer Schraubenfeder realisiert. Die optische Einheit ist nicht unmittelbar am Probenblock justiert. Die direkte Anbringung der Lichtleitfaser im Probenblock garantiert einen minimalen Weg des Lichtes nach Austritt aus der Faser und dem Übergang in die Probe (Minimierung von Streuungs- und Brechungseffekten). Es ist daher nicht möglich, das erfindungsgemäße Consumable in der im Dokument US 6,565,815 B1 beschriebenen Vorrichtung zu fahren/betreiben und umgekehrt. Zur optimalen Wärmeübertragung in die Probe ist ein gewisser Anpressdruck der Folien der Reaktionskammer gegen den Probenblock nötig. Dies wird erfindungsgemäß durch die Anpresskraft und Formschlüssigkeit der Probenblöcke zur Reaktionskammer und dem damit verbundenen abdichten der Probenkammer realisiert (Abklemmen des Injektionskanals). Auch hierbei zeigen sich die Vorteile der erfindungsgemäß konvex ausgeformten Probenblöcke.

Die erfindungsgemäße Vorrichtung unterscheidet sich auch von der, die in DE 10 2006 036 171 A1 beschrieben wurde, deutlich. Der Hauptunterschied besteht hier im Abstand der Fasern zur Probe. Das Licht muss in DE 10 2006 036 171 A1 erst die Sealingfolie der Proben und dann den Headspace über der Probe durchdringen bis es in die Probe gelangt. Dies führt wiederum zu erheblichen Streuungs- und Brechungseffekten, die in der erfindungsgemäßen Anordnung nicht auftreten.

Das erfindungsgemäße Verfahren der dualen Detektion läuft dabei nach folgenden Schritten ab:

### A. Bereitstellung eines Reaktionsansatzes, bestehend aus:

◆ einer eine Nukleinsäure enthaltenden Probe, in der die Target-Nukleinsäure nachgewiesen werden soll
◆ mindestens einem durch eine Markierung 1 versehenes Oligonukleotid, das vollständig oder teilweise zu der Targetsequenz komplementär ist und als ein Primer in einer matrizenabhängige Neusynthese der Targetnukleinsäure fungiert (Oligo-Typ 1)
◆ mindestens einem durch eine Markierung 2 markierten Oligonukleotid, das aufgrund der niedrigeren Schmelztemperatur als Oligonukleotid 1 nicht am DNA-Neusyntheseprozess beteiligt ist, aber mit dem DNA-Neusyntheseprodukt von Oligo Typ 1 teilweise oder vollständig hybridisieren kann (Oligo Typ 2)
◆ einem Chemikalien-/Enzym-Gemisch ggf. auch mit weiteren, nicht markierten Oligonukleotiden, das eine matrizenabhängige Neusynthese der Targetnukleinsäure ermöglicht.

Erfindungsgemäß werden die Markierungen der beiden Oligonukleotide (Oligo Typ 1 und Oligo Typ 2) so ausgewählt, dass sie zusammen ein FRET-Paar bilden (z.B. FITC/TAMRA, FAM/TAMRA, FAM/BHQ1 u.ä.) und in Bezug auf die erfindungsgemäße Doppeldetektion auch komplementäre Bindungspartner auf einem Lateral-Flow-Streifen haben können.

### B. Durchführung der matrizenabhängigen DNA-Neusynthese mit integrierter Sondenhybridisierung

**In** der Abhängigkeit von der Art der Target-Nukleinsäure wird entweder eine Reverse Transkriptase Reaktion (bei RNA, die in einer sehr großen Kopienzahl vorkommt z.B. rRNA, tmRNA) oder eine Amplifikation (bei DNA) oder auch die beiden Reaktionen nacheinander folgend (bei einer raren RNA z.B. mRNA, Proben mit einer geringen Partikelzahl) durchgeführt.

ln dieser ersten Reaktion ist auf Grund des erfindungsgemäßen Verfahrens nur das Oligo Typ 1 beteiligt. Das Oligo Typ 1 fungiert als ein Primer entweder in einer RNA-abhängigen Reversen Transkription (dadurch entsteht ein markierter cDNA-Strang) oder in einer Amplifikation der Target-DNA bzw. cDNA (dadurch entsteht ein markiertes PCR-Produkt). OneStep RT-PCR kann gleichfalls durchgeführt werden. Dabei erhöht ein zweites, nichtmarkiertes Primer-Oligonukleotid die Ausbeute der PCR-Reaktion.

Das Oligo Typ 2 ist auf Grund seiner niedrigeren Anealingtemperatur gemäß dem erfindungsgemäßen Verfahrens nicht an der DNA-Neusynthese beteiligt. Nachfolgend wird der Reaktionsansatz erhitzt bei einer Temperatur > 90°C. Dieser Schritt führt zur thermischen Strangtrennung. Nach dem Ablauf dieser Denaturierungsreaktion wird der Reaktionsansatz auf die Hybridisierungstemperatur des Oligos Typ 2 abgekühlt. Während dieses Schrittes bindet das Oligo Typ 2 spezifisch an den komplementären DNA-Strang. Dieser Strang trägt dabei die Markierung 1, welche durch das Oligo Typ 1 in das Reaktions-Produkt eingebaut wurde.

### C. Dualer Nachweis des Hybridisierungsereignisses

Die Nachweisreaktion erfolgt in derselben Reaktionskartusche in nacheinander ab.
1. Nachweis der Hybridisierungsreaktion mittels einer Fluoreszenzmessung Die durch die von den beiden Oligos Typ 1 und Typ 2 eingebauten Markierungen bilden ein FRET-Paar. Die im erfindungsgemäßen Verfahren erfolgende Hybridisierung des Oligos Typ 2 an das Syntheseprodukt des Oligos Typ 1 führt zu einem FRET-Effekt zwischen der Markierung 1 und 2. Dieser Effekt führt jetzt zu einer messbaren Verringerung der Fluoreszenz. Diese Reduzierung der Fluoreszenz wird numerisch ausgewertet und ermöglicht so den eindeutigen Nachweis der Reaktion. Nach der erfolgten optischen Detektion wird der Reaktionsansatz durch die Zugabe eines Laufpuffers, welcher in die Reaktionskartusche injiziert wurde, in die zweite Reaktionskammer der Kartusche (dort befindet sich ein Lateral Flow Streifen) überführt. Der Transfer des Reaktionsansatzes/Laufpuffers wird über die Fläche realisiert, welche durch die den Basiskörper der Reaktionskartusche abschließenden Folie erzeugt wurde
2. Nachweis der Hybridisierungsreaktion auf dem Lateral Flow Streifen in der zweiten Reaktionskammer der erfindungsgemäßen Reaktionskartusche

Der Lateral-Flow-Streifen enthält eine Bindungsstelle für eine der Markierungen der Oligos Typ 1 oder Typ 2 und / oder Antikörper oder andere Bindungsmoleküle gegen die Markierungsmoleküle der Oligos Typ1 oder Typ 2, welche an die Markierungsmoleküle Typ 1 oder Typ 2 binden können (z.B. kovalente Bindungen oder Wasserstoffbrückenbindung oder über Brückenmoleküle). Darüber hinaus befindet sich auf der festen Phase ein Nachweismolekül für eine Visualisierung bzw. Messung des Hybridisierungsereignisses oder ein solches Nachweismolekül wird der Detektionsreaktion zugeführt. Das Nachweismolekül kann aber auch schon während der Amplifizierungs-/ Hybridisierungsreaktion in das nachzuweisende Hybridisierungsprodukt eingebaut worden sein.

Der Nachweis der immunologischen Reaktion auf dem Teststreifen erfolgt dann einfach, z.B. auf visuellem Wege.

Nach Befundung wird die Reaktionskartusche verworfen.

Zusammenfassend steht mit dem erfindungsgemäßen Verfahren nun ein extrem einfaches und universell einsetzbares gendiagnostisches Nachweisverfahren zur Verfügung.

Die duale Detektion einer nachzuweisenden diagnostisch relevanten Targetnukleinsäure erfolgt in Form eines homogenen Assays erfindungsgemäß über die Endpunkt-Fluoreszenzmessung einer Fluoreszenzlöschung und nachfolgenden über die immunologischen Detektion auf einem Lateral Flow Streifen. Das Ergebnis kann numerisch erfasst werden und erlaubt auch eine Detektion und Quantifizierung der nachzuweisenden Targetnukleinsäure (unter Verwendung eines internen Standards). Das erfindungsgemäße Verfahren der dualen Detektion realisiert damit in eleganter und extrem einfacher Art und Weise einen ersten diagnostischen Nachweis einer Zielnukleinsäure mittels Fluoreszendetektion und nachfolgend in der selben Reaktionskartusche eine Ergebnisverifizierung auf einem Lateral-Flow-Streifen.

Eine solche Verifizierung ist damit diagnostisch sehr viel exakter, als ein bisher möglicher Nachweis von RealTime-PCR-Produkten auf einem Agarosegel. Das Verfahren kann auch unter Feldbedingungen eingesetzt werden.

Diese elegante neuartige Testdurchführung, insbesondere auch die kombinierte Testdurchführung (Fluoreszenznachweis und nachfolgende Verifizierung der ersten Testreaktion an einer festen Phase) werden erfindungsgemäß dadurch ermöglicht, dass während der Amplifikation und Hybridisierung die hybridisierte Sonde (Oligo Typ 2) nicht durch die Taq-Polymerase abgebaut wird, sondern auch nach dem Reaktionsablauf im hybridisierten Zustand bleibt im Gegensatz zum homogenen TaqMan-Exonuklease-Assay.

Die erfindungsgemäße Integration einer Hybridisierungssonde in die Reaktion gibt die Sicherheit, dass das amplifizierte Fragment wirklich die Zielsequenz beinhaltet. Dadurch werden die durch Mispriming entstehenden falschpositiven Resultate ausgeschlossen. Die Verwendung der chemisch modifizierten Sonde (vorzugsweise Phosphorylierung des letzten Nukleotides der Sonde) verhindert die Verlängerung der Sonde durch 5'-->3'-Polymeraseaktivität und verhindert damit, dass die Sonde als Primer fungiert und unspezifische PCR-Artefakte (Primer-Dimere) erzeugt, die als falsch positive Signale detektiert werden würden.

Im Unterschied zu RealTime-PCR-Verfahren erfolgt die Detektion des spezifischen Nachweissignals nicht während der Amplifikation, wo die Fluoreszenz entweder durch die von der Taq-Polymerase durchgeführten Sondenhydrolyse freigesetzt wird (EP 0972 848 A2) oder durch den FRET-Effekt vermindert wird (EP1384789 B1), sondern erst nach dem Abschluss der Amplifizierungs-Hybridisierungs-Reaktion. Ein solches Verfahren würde auch eine nachfolgende immunologische Detektion auf einem Lateral-Flow-Teststreifen nicht erlauben.

Das erfindungsgemäße Verfahren unterscheidet sich auch von der Patentschrift (EP 0 826 066 B1), welche ebenfalls eine Kombination von PCR und Hybridisierung darstellt. Auch bei diesem Verfahren wird wiederum ein durch FRET-Effekt vermitteltes Fluoreszenzsignal detektiert. Dieses entsteht während des Amplifikationsvorganges durch die Hybridisierung einer doppeltmarkierten Sonde, die eine niedrigere Annealingtemperatur besitzt als die Primer. Die Freisetzung der Fluoreszenz erfolgt dabei nicht durch Hydrolyse der Sonde infolge der Exonukleaseaktivität der Polymerase, sondern dadurch, dass bei der Hybridisierung die sekundäre Struktur der Sonde aufgelöst wird und durch die Entfernung des Reporters von dem Quencher die Fluoreszenz freigesetzt wird. Hierbei können nur Enzyme für die Amplifikation eingesetzt werden, die keine Exonukleaseaktivität besitzen (z.B. Klenow Fragment oder T4 oder T7 Polymerasen).

Wie schon aufgeführt, kann das erfindungsgemäße Verfahren auch unter Feldbedingungen durchgeführt werden. Die gerätetechnische Basis wurde bereits in den Druckschriften DE102007062441A1 und WO2009/080817A2 offenbart. Das erfindungsgemäße neuartige Verfahren der dualen Detektion und die erfindungsgemäße verbesserte Reaktionskartusche zeichnet sich ferner dadurch aus, dass sie es ermöglicht, innerhalb der Reaktionsgefäße eine schnelle PCR, die so genannte rapid PCR durchzuführen. Bei dieser Form der PCR müssen einem Reaktionsgefäß bzw. Reaktionsraum sehr schnell große Energiemengen zugeführt werden um sehr schnelle Temperaturänderungen zu erzielen (bis zu 15°C/s).

Dies setzt bei Standard-Consumables bzw. -Gefäßen voraus, dass sehr starke Heizelemente (Peltierelemente, Heizfolien usw.) zum Einsatz kommen und somit, aufgrund ihrer Leistungsaufnahme, einem mobilen Einsatz aufgrund der benötigten limitierenden Batterien bzw. Akkumulatoren, widersprechen.

Um diese Problematik zu umgehen, wird z.B. bei bestehenden Chiplösungen das zu beheizende Volumen sehr weit herabgesetzt (<1µl bis zu nl Volumen), um hier nur ein geringes Volumen heizen zu müssen. Hierbei wird jedoch die Handhabbarkeit eines solchen Volumens völlig außer Acht gelassen. Ferner treten hier erhebliche Verdunstungs-Problematiken bei einem solchen Volumen auf.

Erstaunlicherweise konnte schon bei den ersten Versuchen, bei der Verwendung der erfindungsgemäßen Kartusche, gezeigt werden, dass auch mit der Verwendung von handelsüblichen Standard Peltierelementen eine extrem hohe Heizrate in einem großen Volumen (25µl) erzielt werden konnte. Dies wurde durch die Verwendung der abschließenden Folien auf dem Basiskörper erzielt. Diese Folien können - ohne einen Stabilitätsverlust der Gesamtkartusche zu verursachen - in minimalen Dicken gewählt werden (<100µm).

Die abschließenden Folien bilden mit dem Basiskörper einen Reaktionsraum, welcher mit dem Reaktionsansatz gefüllt werden kann. Um den benötigten Wärmeeintrag in das Reaktionsvolumen zu realisieren, werden 2 Probenblöcke (von jeder Seite einer Figur 2) mit einer großen Kraft (z.B. jeweils >60N) auf den Reaktionsraum gedrückt. Die Probenblöcke weisen dabei eine leicht in den Probenblock gewölbte Form auf, und schließen sie durch die Form und die große Kraft hermetisch ab. Die ausgeübte Kraft der Probenblöcke kann sehr hoch gewählt werden, da die abschließende Folie nur leicht gewölbt wird und sie, auch bei minimaler Dicke der Folie, nicht zerstört wird.

Durch die Wölbung der Probenblöcke bildet sich in der Mitte der Reaktionskammer eine leicht verengte Stelle aus. Durch die senkrechte Anordnung der Kartusche wird die Problematik mit entstehenden Luftblasen in der Probenflüssigkeit gelöst, da sich die Luftblasen im oberen Teil der Reaktionskammer zum einem Luft gefüllten Raum ausbilden. Diese Problematik findet sich in vielen state of the art Systemen wieder und wird teilweise sehr aufwendig vermieden (Luftblasen in der Probenflüssigkeit). In der erfindungsgemäßen Anordnung der Kartusche wirkt dieser sich ausbildende luftgefüllte Raum sogar positiv aus. Erstaunlicherweise zeigte sich in der Ermittlung der Heizraten, dass wenn sich ein Luftraum gebildet hat, der Druck im Gefäß ansteigt (Ausdehnung der Luft). Der ansteigende Druck bei hohen Temperaturen (z.B. 90°C) vermag es, dass die Folie für einen noch besseren Wärmeübergang an die Probenblöcke gedrückt wird und verbessert somit nochmals die Wärmeübertragung. Dieser Effekt kann bei diesem Consumable genutzt werden, ist jedoch für die erfindungsgemäße Verwendung nicht ausschlaggebend.

In der erfindungsgemäßen Gerätekonfiguration befindet sich in Erweiterung zu den offenbarten Gerätesystem der o.g. Druckschriften jetzt ein optische Detektionseinheit.

Die optische Detektion ermöglicht die beschriebene Messung der Fluoreszenz für die erste Nachweisreaktion in der ersten Reaktionskammer der Reaktionskartusche:

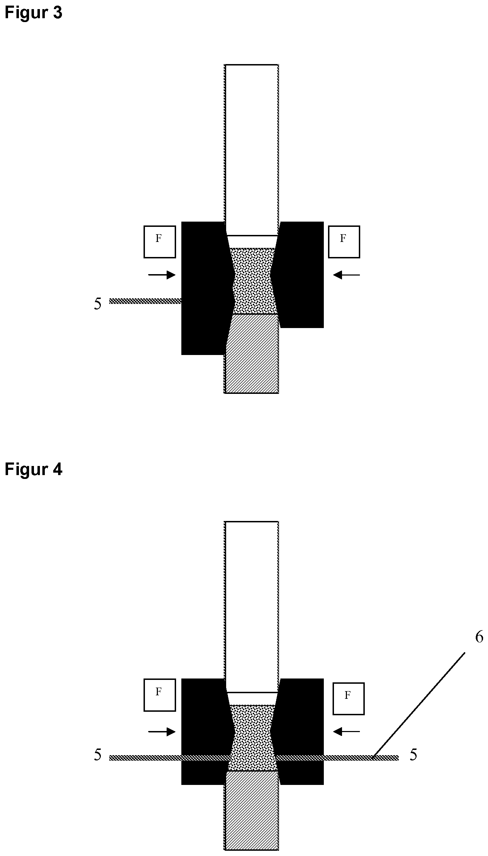

Der Abstand der abschließenden Folien zueinander wird nach dem Anpressen der Probenblöcke durch die Dicke des Basiskörpers und die Geometrie der Probenblöcke bestimmt. Diese reproduzierbare Distanz zwischen den Probenblöcken zeigte sich in Testmessungen als eine sehr gute Grundlage für eine optische Detektion innerhalb einer Reaktionskammer. Eine optische Weglänge wird also nur durch die Dicke des Consumables definiert (bei konstanter Anordnung der Probenblöcken). Dieser Effekt erlaubt es, eine reproduzierbare Absorbtionsmessung von einem Probenblock zum anderen durchzuführen, um beispielsweise einen Nachweis über eine DNA-Konzentration führen zu können (siehe Figur 3 und Figur 4 Reaktionskammer mit optischer Detektion bzw. Anordnungen zur Fluoreszenzdetektion und Absorptionsmessung durch die Reaktionskammer). Dazu wird an einem Probenblock Licht in die Kartusche (durch die abschließende Folie) eingebracht (z.B. über Lichtleitfaser) und im anderen Probenblock durch die abschließende Folie (z.B. ebenfalls über eine Lichtleitfaser) einem Detektor zugeführt. Die abschließenden Folien werden hierbei natürlich so ausgewählt, dass sie den optischen Anforderungen der Detektionsmethode entsprechen (z.B. wenig Absorption des Lichtes durch die abschließenden Folien / die abschließenden Folien müssen durchgängig für Wellenlängen des Detektionsprinzips sein).

Die Probenblöcke liegen bei nicht eingesetzter Kartusche mit Ihrer Wölbung direkt aufeinander, wobei die Anpresskraft z.B. durch einen Federmechanismus bzw. einen einfachen mechanischen Aufbau realisiert werden kann. Wird nun eine Kartusche mit einer unterschiedlichen Dicke des Basiskörpers eingesetzt, wird die optische Weglänge beeinflusst, jedoch nicht die Amplifikation der Probe durch die rapid PCR. Der Wärmeübergang durch die abschließenden Folien macht eine komplizierte Regelung der Temperaturführung überflüssig, da die Temperatur in den Probenblöcken fast 100% der in der Kartusche entspricht. Durch die Dickenänderung des Basisträgers können somit das Volumen der Reaktionskammer und die optische Weglänge positiv beeinflusst werden.

Es ist ebenfalls möglich, in einem Probenblock (z.B. über mindestens eine Lichtleitfaser) eine Anregungswellenlänge in die Kartusche zu leiten und über die identische Faser (oder eine 2. Faser, ebenfalls im gleichen Probenblock oder im 2. Probenblock befindlich) die Fluoreszenzantwort der Probe einem Detektor zuzuführen (siehe Figur 3: Reaktionskammer mit optischer Detektion bzw. Anordnungen zur Fluoreszenzdetektion).

Die Anordnung erlaubt es ebenfalls, beide Detektionen in einer Anordnung zu vereinen, indem beide Probenblöcke für Absorption und Fluoreszenzdetektion ausgelegt sind (siehe auch Reaktionskammer mit optischer Detektion bzw. Anordnungen zur Fluoreszenzdetektion und Absorptionsmessung durch die Reaktionskammern).

In der Kombination des erfindungsgemäßen Verfahrens zur dualen Detektion einer Zielnukleinsäure mit der erfindungsgemäßen Vorrichtung steht damit erstmals ein Testsystem zur Verfügung, das es auch unter Feldbedingungen erlaubt, ein erstes diagnostisches Ergebnis durch ein zweites diagnostisches Testergebnis zu bestätigen. Damit kann die Sicherheit einer diagnostischen Aussage deutlich verbessert werden.

Die erfindungsgemäße Kartusche besteht aus mindestens einem Basiskörper, mindestens einer ihn abschließenden Folie, welche mindestens eine nicht mit dem Basiskörper verbundene Fläche aufweist, welche mindestens ein Volumen mit dem Basiskörper zum Medientransfer und/oder mindestens eine Reaktionskammer ausbildet.

Die mindestens eine ausgebildete Reaktionskammer hat folgende Vorteile:
◆ Die Detektion eines oder mehrerer Targets ist möglich
◆ Eine optische Detektion einer Amplifikation eines oder mehrerer Targets kann durchgeführt werden
◆ Eine Detektion mittels eines Teststreifens eines oder mehrerer Targets kann durchgeführt werden
◆ Jeweils ein sich unterscheidendes Detektionsverfahren eines oder mehrerer Targets kann durchgeführt werden.
◆ 2 verschiedene Detektionsmethoden sind möglich

### Legende zu den Figuren

Figur 1 zeigt einen Ausschnitt des Reaktionsraums mit Folien.
Figur 2 zeigt einen Ausschnitt der Reaktionskammer mit Probenblöcken und Probenblöcke ohne Reaktionskammer.
In Figur 3 sind die Reaktionskammer mit optischer Detektion bzw. Anordnungen zur Fluoreszenzdetektion zu erkennen.
   Die Reaktionskammer mit optischer Detektion bzw. Anordnungen zur Fluoreszenzdetektion und Absorptionsmessung durch die Reaktionskammer sind in Figur 4 abgebildet.
Figur 5 zeigt die Basisanordnung der Reaktionskartusche.
Figur 6 zeigt die Ausbildung von nicht verbundenen Flächen der Folie mit dem Basiskörper zum Volumentransfer (Darstellung ohne Volumentransfer).
Figur 7 zeigt die Ausbildung von nicht verbundenen Flächen der Folie mit dem Basiskörper zum Volumentransfer (Darstellung mit Volumentransfer). Das Bezugszeichen 16 zeigt den Volumentransfer durch die nicht verbundene Fläche der Fläche zum Basisträger (wird Flüssigkeit unter Druck in die nicht verbundene Fläche gedrückt, wölbt sich die Folie und ein Volumentransfer wird ermöglicht).

### Bezugszeichenliste

Die Bezugszeichen bedeuten:

| | | | |
|---|---|---|---|
| 1 | Basiskörper | 2 | Reaktionsvolumen |
| 3 | abschließende Folie 1 | 4 | abschließende Folie 2 |
| 5 | Optische Bauelemente / Lichtquelle und/oder Detektor | | |
| 6 | Lichtleitfaser | 7 | Teststreifen (lateral flow stripe) |
| 8 | Kammer für Teststreifen | 10 | Reaktionskammer |
| 9 | Strömungskanal zur Kammer mit Teststreifen | | |
| 11 | Strömungskanal zur Reaktionskammer | | |
| 12 | Eingabeöffnung für Medieneintritt | | |
| 13 | Nicht verbundene Fläche der Folie mit dem Basiskörper | | |
| 14 | Verbundene Flächen der Folie mit dem Basiskörper | | |
| 15 | Basiskörper | 16 | Volumentransfer |

## Patentansprüche

1. Vorrichtung zum Nachweis von Nukleinsäuren, umfassend mindestens zwei beheizbare Probeblöcke und eine Reaktionskartusche, die einen Basiskörper (1,15) und mindestens eine den Basiskörper (1,15) abschließende Folie (3,4) enthält, **dadurch gekennzeichnet, dass** die Folie (3,4) außerdem nicht mit dem Basiskörper verbundene Flächen (13) aufweist und diese Flächen (13) mindestens zwei Reaktionskammern (8,10) ausbilden, wobei die eine Reaktionskammer (10) eine optische Detektionseinheit zum Nachweis von Nukleinsäuren aufweist und sich in der zweiten Reaktionskammer (8) ein Teststreifen (7) zum Nachweis von Nukleinsäuren befindet.

2. Vorrichtung nach Anspruch 1, **dadurch gekennzeichnet, dass** die Probeblöcke eine Wölbung haben und dadurch eine Verengung in mindestens einer Reaktionskammer gebildet wird.

3. Vorrichtung nach Anspruch 1, **dadurch gekennzeichnet, dass** die Dicke der Folie kleiner als 100µm ist.

4. Vorrichtung nach Anspruch 1, **dadurch gekennzeichnet, dass** die Reaktionskartusche senkrecht zu den Probeblöcken angeordnet ist.

5. Vorrichtung nach Anspruch 1, **dadurch gekennzeichnet, dass** der Abstand der abschließenden Folie zueinander nach dem Anpressen der Probenblöcke durch die Dicke des Basiskörpers und die Geometrie der Probenblöcke bestimmt wird.

6. Vorrichtung nach Anspruch 1, **dadurch gekennzeichnet, dass** die Probenblöcke bei nicht eingesetzter Kartusche mit Ihrer Wölbung direkt aufeinander liegen, wobei die Anpresskraft durch einen Federmechanismus realisiert wird.

7. Vorrichtung nach Anspruch 1, **dadurch gekennzeichnet, dass** sich in der zweiten Reaktionskammer ein Lateral-Flow-Streifen zum immunologischen Nachweis einer Amplifikations- /Hybridisierungsreaktion bei Nukleinsäuren befindet.

8. Vorrichtung nach Anspruch 1, **dadurch gekennzeichnet, dass** an einem Probenblock sich ein Gerät zum Einbringen von Licht in die Kartusche durch die abschließende Folie, vorzugsweise mittels einer Lichtleitfaser, befindet und sich im anderen Probenblock ein Durchlass für dieses Licht zu einem Detektor befindet.

9. Vorrichtung nach Anspruch 1, **dadurch gekennzeichnet, dass** in einem Probenblock ein Gerät zum Einbringen einer Anregungswellenlänge mittels einer Lichtleitfaser in die Kartusche befindet und über die identische Faser oder eine 2. Faser, im gleichen oder anderen Probenblock ein Durchlass zu einem Detektor befindet, der eine Fluoreszenzantwort der Probe registriert.

10. Vorrichtung nach Anspruch 1, **dadurch gekennzeichnet, dass** die zwei Probenblöcke mit einer Kraft von mindestens 5N, vorzugsweise mindestens 30N, auf den Reaktionsraum gedrückt werden können.

11. Verfahren zum Nachweis von Nukleinsäuren, **gekennzeichnet durch** folgende Schritte:
a) Bereitstellung eines Reaktionsansatzes, bestehend aus:
• einer eine Nukleinsäure enthaltenden Probe, in der die Target-Nukleinsäure nachgewiesen werden soll
• mindestens einem durch eine Markierung 1 versehenes Oligonukleotid, das vollständig oder teilweise zu der Targetsequenz komplementär ist und als ein Primer in einer matrizenabhängige Neusynthese der Targetnukleinsäure fungiert (Oligo-Typ 1)
• mindestens einem durch eine Markierung 2 markierten Oligonukleotid, das aufgrund der niedrigeren Schmelztemperatur als Oligonukleotid 1 nicht am DNA-Neusyntheseprozess beteiligt ist, aber mit dem DNA-Neusyntheseprodukt von Oligo Typ 1 teilweise oder vollständig hybridisieren kann (Oligo Typ 2)
• einem Chemikalien-/Enzym-Gemisch ggf. auch mit weiteren, nicht markierten Oligonukleotiden, das eine matrizenabhängige Neusynthese der Targetnukleinsäure ermöglicht.
b) Durchführung einer matrizenabhängigen DNA-Neusynthese mit integrierter Sondenhybridisierung
c) Dualer Nachweis des Hybridisierungsereignisses, wobei in einer Reaktionskammer der Vorrichtung gemäß einem der Ansprüche 1 bis 10 ein optischer Nachweis und in einer zweiten Reaktionskammer der Vorrichtung gemäß einem der Ansprüche 1 bis 10 ein Nachweis auf einem Lateral Flow Streifen erfolgt.

12. Verfahren nach Anspruch 11, **dadurch gekennzeichnet, dass** der Lateral-Flow-Streifen eine Bindungsstelle für eine der Markierungen der Oligos Typ 1 oder Typ 2 und / oder Antikörper oder andere Bindungsmoleküle gegen die Markierungsmoleküle der Oligos Typ1 oder Typ 2 enthält, die an die Markierungsmoleküle Typ 1 oder Typ 2 binden können.

## Claims

1. A device for detecting nucleic acids comprising at least two heatable sample blocks and a reaction cartridge containing the base (1, 15) and at least one film (3, 4) sealing the base (1, 15) **characterized in that** the film (3, 4) moreover comprises surfaces (13) that are not connected to the base and said surfaces (13) form at least two reaction chambers (8, 10), wherein the one reaction chamber (10) comprises an optical detection unit for the detection of nucleic acids and a test strip (7) is located in the second reaction chamber (8) for the detection of nucleic acids.

2. The device according to claim 1, **characterized in that** the sample blocks have a curvature and thus a narrowing in at least one reaction chamber is formed.

3. The device according to claim 1, **characterized in that** the thickness of the film is smaller than 100µm.

4. The device according to claim 1, **characterized in that** the reaction cartridge is located perpendicular to the sample blocks.

5. The device according to claim 1, **characterized in that** the distance of the sealing film to each other after pressing-on of the sample blocks is determined by the thickness of the base and the geometry of the sample blocks.

6. The device according to claim 1, **characterized in that** the sample blocks rest on one another with their curvature when the cartridge is not inserted, wherein the pressing force is realized by a spring mechanism.

7. The device according to claim 1, **characterized in that** a lateral-flow-strip for immunological detection of an amplification / hybridization reaction in nucleic acids is located in the second reaction chamber.

8. The device according to claim 1, **characterized in that** an apparatus for the introduction of light into the cartridge through the sealing film, preferably by means of an optical fiber, is located on a sample block, and a passageway for said light to a detector is located in another sample block.

9. The device according to claim 1, **characterized in that** in a sample block an apparatus for the introduction of an excitation wavelength into the cartridge by means of an optical fiber is located, and via the identical fiber or a second fiber in the same or in another sample block a passageway to a detector is located which registers a fluorescence response of the sample.

10. The device according to claim 1, **characterized in that** the two sample blocks can be pressed onto the reaction space by means of a force of at least 5N, preferably at least 30N.

11. Method for detecting nucleic acids, **characterized by** the following steps:
a) providing a reaction batch consisting of:
• a sample containing a nucleic acid in which the target nucleic acid is to be detected
• at least an oligonucleotide provided by a label 1, which is wholly or partially complementary to the target sequence and acts as a primer in a matrix-dependent new synthesis of the target nucleic acid (olio type 1)
• at least an oligonucleotide labeled by a label 2 which, due to the lower melting temperature than oligonucleotide 1 is not involved in the DNA new synthesis process but can partly or wholly hybridize with the DNA new synthesis product of oligo type 1 (oligo type 2)
• a chemicals/enzyme mixture, if required, also with further non-labeled oligonucleotides permitting a matrix-dependent new synthesis of the target nucleic acid
b) carrying out a matrix-dependent DNA new synthesis with integrated probe hybridization
c) dual detection of the hybridization event, wherein in a reaction chamber of the device according to any one of claims 1 to 10 an optical detection and in a second reaction chamber of the device according to any one of claims 1 to 10 a detection on a lateral flow strip occurs.

12. Method according to claim 11, **characterized in that** the lateral flow strip contains a binding site for one of the labels of the oligos type 1 or type 2 and / or antibodies or other binding molecules against the labeling molecules of the oligos type 1 or type 2 which can bind to the labeling molecules type 1 or type 2.

## Revendications

1. Dispositif de détection d'acides nucléiques comportant au moins deux blocs d'échantillons chauffables et une cartouche de réaction contenant un corps de base (1, 15) et au moins un film (3, 4) fermant le corps de base (1, 15), **caractérisé en ce que** le film (3, 4) comporte en outre des surfaces (13) qui ne sont pas liées avec le corps de base et ces surfaces (13) forment au moins deux chambres de réaction (8, 10), une chambre de réaction (10) comportant une unité de détection optique pour détecter des acides nucléiques et une bande de test (7) se trouvant dans la seconde chambre de réaction (8) pour détecter des acides nucléiques.

2. Dispositif selon la revendication 1, **caractérisé en ce que** les blocs d'échantillons présentent une courbure et ainsi un rétrécissement est formé dans au moins une chambre de réaction.

3. Dispositif selon la revendication 1, **caractérisé en ce que** l'épaisseur du film est inférieure à 100 µm.

4. Dispositif selon la revendication 1, **caractérisé en ce que** la cartouche de réaction est agencée perpendiculairement aux blocs d'échantillons.

5. Dispositif selon la revendication 1, **caractérisé en ce que** la distance du film fermant entre eux après le pressage des blocs d'échantillons est déterminée par l'épaisseur du corps de base et la géométrie des blocs d'échantillons.

6. Dispositif selon la revendication 1, **caractérisé en ce que** les blocs d'échantillons, lorsque la cartouche n'est pas insérée, restent directement l'un sur l'autre avec leur courbure, la force de pression étant réalisée par un mécanisme à ressort.

7. Dispositif selon la revendication 1, **caractérisé en ce qu'**un bande d'écoulement latérale (Lateral-Flow) se trouve dans la seconde chambre de réaction pour la détection immunologique d'une réaction d'amplification / de hybridation aux acides nucléiques.

8. Dispositif selon la revendication 1, **caractérisé en ce que** sur un bloc d'échantillons il se trouve un appareil d'introduction de la lumière dans la cartouche à travers le film fermant, de préférence au moyen d'une fibre optique, et dans l'autre bloc d'échantillons il se trouve un passage pour cette lumière à un détecteur.

9. Dispositif selon la revendication 1, **caractérisé en ce que** dans un bloc d'échantillons il se trouve un appareil d'introduction d'une longueur d'onde d'excitation au moyen d'une fibre optique dans la cartouche et via la fibre identique ou une seconde fibre dans le même bloc d'échantillons ou dans un autre bloc d'échantillons il se trouve un passage à un détecteur qui enregistre une réponse de fluorescence de l'échantillon.

10. Dispositif selon la revendication 1, **caractérisé en ce que** les deux blocs d'échantillons peuvent être pressés sur l'espace de réaction avec une force d'au moins 5N, de préférence au moins 30N.

11. Procédé de détection d'acides nucléiques, **caractérisé par** les étapes suivantes:
a) fournir une préparation de réaction comportant
• un échantillon contenant un acide nucléique dans lequel l'acide nucléique cible doit être détecté
• au moins un oligonucléotide pourvu d'un marquage 1 qui est entièrement ou partiellement complémentaire à la séquence cible et agit en tant que *primer* dans une resynthèse dépendante de la matrice d'acide nucléique cible (oligo type 1)
• au moins un oligonucléotide marqué par un marquage 2 qui en vertu de la température de fusion inférieure à l'oligonuclétoide 1 ne participe pas au processus de resynthèse ADN mais peut hybrider entièrement ou partiellement avec le produit de la resynthèse ADN d'oligo type 1 (oligo type 2)
• un mélange des produits chimiques/enzymes, le cas échéant, aussi avec d'autres oligonucléotides non marqués permettant une resynthèse dépendante de la matrice de l'acide nucléique cible.
b) réaliser un resynthèse ADN dépendante de la matrice avec une hybridation de sonde intégré
c) détection duale de l'événement d'hybridation, une détection optique ayant lieu dans une chambre de réaction du dispositif selon l'une quelconque des revendications 1 à 10 et dans une seconde chambre de réaction du dispositif selon l'une quelconque des revendications 1 à 10 une détection ayant lieu sur une bande d'écoulement latérale (Lateral Flow).

12. Procédé selon la revendication 11, **caractérisé en ce que** la bande d'écoulement latérale (Lateral Flow) comporte un site de liaison pour un des marquages de l'oligo type 1 ou type 2 et / ou des anticorps ou d'autres molécules de liaison contre les molécules de marquage des oligos type 1 ou type 2 qui peuvent lier aux molécules de marquages type 1 ou type 2.
